Europäisches Patentamt

(19) European Patent Office　　　　　(11) Publication number:　　**0 138 564**

Office européen des brevets　　　　　　　　　　　　　　　　**B1**

⑫　　　　　　　　　　**EUROPEAN PATENT SPECIFICATION**

(45) Date of publication of patent specification: **07.01.88**　　(51) Int. Cl.⁴: **A 61 F 9/00**

(21) Application number: **84306895.8**

(22) Date of filing: **10.10.84**

(54) Myopia alleviation prosthesis.

(30) Priority: **11.10.83 US 540344**

(43) Date of publication of application:
**24.04.85 Bulletin 85/17**

(45) Publication of the grant of the patent:
**07.01.88 Bulletin 88/01**

(84) Designated Contracting States:
**AT BE CH DE FR GB IT LI LU NL SE**

(56) References cited:
**WO-A-81/00570**
**FR-A-2 354 089**
**FR-A-2 354 752**
**SU-A- 596 237**
**SU-A- 628 913**
**SU-A- 797 685**
**SU-A- 997 675**
**US-A-4 299 227**
**MEDICAL & BIOLOGICAL ENGINEERING &
COMPUTING, vol. 15, no. 5 September 1977,
pages 564-572, M.S. ENGLEMAN et al.:
"Computer localisation of detached retinal
tears for a scleral buckling procedure"**
**American Journal of Ophtalmology,86, 1978,
pages 782-790, F.B. Thompson: "A simplified
scleral reinforcement technique"**

(73) Proprietor: **White, Thomas C.**
**1127 Holly Drive**
**Sioux Falls South Dakota 57105 (US)**

(72) Inventor: **White, Thomas C.**
**1127 Holly Drive**
**Sioux Falls South Dakota 57105 (US)**

(74) Representative: **Parr, Ronald Edward R.E. Parr &
Co.**
**Colman House Station Road**
**Knowle Solihull West Midlands B93 0HL (GB)**

Courier Press, Leamington Spa, England.

## Description

### Field of the Invention

The invention relates to the field of medicine and particularly to prostheses for improving the vision of myopic patients.

### Background of the Invention

A patient with normal vision is able to appropriately change the shape of the natural lens so as to focus an image upon the retina, and particularly upon the retinal macula. Myopic persons are unable to focus upon distant objects because the image thereof is formed a short distance (often measured in fractions of a millimeter) in front of the macula. There are various causes of myopia, among which may be listed the failure of the natural lens to change shape sufficiently, abnormal corneal curvature, and an abnormally long eye globe measured in the direction of the optical axis.

Eyeglasses commonly are employed to correct myopia of not greater than several diopters. Contact lenses can be used to correct myopia to the extent of perhaps twenty diopters. A surgical procedure known as a radial keratotomy has been employed in recent years to at least partially correct myopia, the procedure involving making radial, partial-thickness incisions in the cornea for the purpose of slightly flattening the cornea. Another surgical procedure, keratomilieusis, involves removal of a partial thickness corneal button, freezing and grinding the innerside of the button to produce increased or decreased concavity, and then reattaching the corneal button, this procedure also tending to flatten the cornea. The surgical procedures thus described depend greatly upon the technical expertise and manual dexterity of the surgeon, and may in some cases lead to scarring of the cornea or to unintentional invasion of the anterior chamber with the consequent danger of infection. Further, a great deal of judgement must be used with respect to the depth, placement and number of radial incisions in the radial keratotomy procedure and in the degree of tissue removal in the keratomilieusis autograft procedure, and perfect results are seldom obtained. Trauma to the eye in the vicinity of the limbus, of course, may result in the onset of glaucoma.

The prostheses and surgical routines referred to above each have, as their object, a refractive correction anterior to the natural lens.

In the conditions known as progressive axial myopia and posterior staphyloma, the enlargement of the scleral and uveal coats of the eye results in stretching, thinning and deterioration of the retinal nerve tissue, which by its nature cannot grow. The interocular pressure in the globe itself tends to cause the globe to bulge out rearwardly. This, in turn, may cause structural changes in the retina leading to optically uncorrectable decreases in vision. In an effort to support the rearward portion of the globe against further distension and traction on the retina, scleral reinforcement techniques have been developed in which a band of scleral tissue is passed about the posterior portion of the globe to strengthen the globe. This procedure is particularly well reported in Frank B. Thompson, *A Simplified Scleral Reinforcement Technique*, Am. J. Opthalmol 86:782—790, 1978. In the reported procedure, a band of scleral tissue terminating in corneal portions and taken from a donor eye was passed posteriorly about the globe of an eye, the corneal end portions being sutured to the globe anteriorly of its equator. Although such slings by themselves may occasionally reduce an ectatic posterior staphyloma, the rearward portion of the globe is at best strengthened to prevent further distension and to limit the progression of posterior enlargement of the globe.

### Brief Description of the Invention

The present invention provides a prosthesis for myopic eyes, including an elongated strip of biologically acceptable material having suturable end portions and which has a length ranging from about 50 to about 75 mm, and characterised by a pressure pad carried by the strip intermediate its length for bearing against the eye wall obverse to the macular with sufficient force applied longitudinally of the optical axis of the eye to displace the macula longitudinally of said axis toward the cornea of the eye thereby to improve the formation of an image on the macula.

The strip can be, for example longitudinally elastic or inextensible. The pressure pad is preferably flexible but substantially incompressible through its thickness. It is preferably carried on the inner surface of the strip.

In use, the prosthesis can be passed posteriorly of the eye globe with the pressure pad in contact with the globe on the obverse side of the eye wall from the macula, and the ends of the strip are sutured to the sclera anteriorly of the equator of the globe. The pressure pad, pressing inwardly upon the posterior portion of the eye wall obverse to the macula, applies sufficient force anteriorly to the globe parallel to the optical axis of the eye so as to force the macula axially (that is, with respect to the optical axis of the eye) inwardly, e.g., the rearward wall of the globe is indented slightly toward the cornea of the eye, thereby shortening the distance between the macula and cornea to improve the formation of an image on the macula and reduce traction on retinal elements, as may be desired. Since the macula need be moved axially inwardly of the globe toward the cornea only a very short distance, commonly measured in fractions of a millimeter for many cases of myopia, the thickness of the pressure pad, the tension in the strip or both, may be varied so that when the prosthesis is in place, the macula is correctly spaced from the cornea. In this manner, a variably controlled indentation of the posterior ocular segment can be accomplished to reduce the axial length of the globe, thereby correcting mild to severe myopia and, as may be desired, reducing traction on retinal elements to prevent structural damage resulting in permanent optically uncorrectable decreases in vision.

Accordingly, normal use of the prosthesis of the invention involves positioning it behind the globe with the pressure pad bearing anteriorly against the obverse side of the eye wall from the macula to force the latter anteriorly to improve the formation of an image on the macula.

Since the macula is spaced only a short distance (about 5 mm) from the optic nerve, and since pressure against the optic nerve should be avoided, the pressure pad preferably has a recessed edge to accommodate free passage of the optic nerve.

If desired, the thickness of the pressure pad may be externally adjusted after positioning the prosthesis, so as to permit adjustment of the axial position of the macula. Normal use of this form of prosthesis of the invention involves positioning the pressure pad behind the globe and thereafter adjusting the thickness of the pressure pad so as axially to position the macula to an appropriate extent so that images from distant objects are focused upon the macula.

In a preferred form of the invention the prosthesis includes a pad comprising an inflatable reservoir and inflation means, typically a thin tube, leading from the reservoir to permit external inflation thereof after surgical positioning of the prosthesis. The reservoir may be inflated with any appropriate fluid such as saline or, preferably, a hardenable polymeric substance. If a non-hardenable fluid such as saline or air is employed, then means must be provided for maintaining the fluid within the reservoir. If a hardenable, normally polymeric substance is employed, then, upon hardening of the substance, the inflation tube may be removed from the device.

In another embodiment, the pressure pad may include an internally threaded annular washer having therewithin an exteriorly threaded bolt or other shaft, the shaft bearing magnetic means enabling exterior rotation thereof. The shaft may be provided with a bar magnet across its width, and rotation of the shaft within the washer may be affected by providing a strong magnet exterior and desirably, anterior to the eye and rotating the magnet about the optical axis of the eye.

Description of the Drawings

Figure 1 is a plan view of a prosthesis of the invention;

Figure 2 is a side view of the prosthesis of Figure 1;

Figure 3 is a view, in plan, of a strip of corneo-scleral tissue from a donor eye;

Figure 4 is a side view of the tissue strip of Figure 3, folded to provide a pressure pad intermediate its ends;

Figure 5 is a broken-away, plan view of a modified device of the invention;

Figure 6 is a cross-sectional view showing another embodiment of the invention;

Figure 7 is a cross-sectional view of a further embodiment of the invention;

Figure 8 is a largely diagrammatic, broken-away top view of an eye to which the prosthesis of the invention has been secured;

Figure 9 is a rear view of the eye shown in Figure 8;

Figure 10 is a bottom view of the eye of Figure 8;

Figure 11 is a broken-away, cross-sectional view of the rear wall of an eye showing a prosthesis of the invention in place;

Figure 12 is a broken-away, plan view of a modification of the invention;

Figure 13 is a cross-sectional view taken along line 13—13 of Figure 12; and

Figure 14 is a broken-away, cross-sectional view showing a suturing method.

Description of the Preferred Embodiment

Referring first to Figures 8—11, proper understanding of the invention can be aided by first identifying the relevant eye structure. The globe of an eye is shown generally as "G" and includes, anteriorly, a cornea "C". Movement of the eye is governed by the operation of six eye muscles of which the medial and lateral rectus muscles are designated "Med. R." and "Lat. R.". The superior and inferior rectus muscles are designated "Sup. R." and "Inf. R.". The superior and inferior oblique muscles are designated "Sup. Obl." and "Inf. Obl.". The optic nerve is designated "O", and, as is shown in Figure 9, inserts into the globe medially of the optical axis "O.A.". With reference to Figure 11, which is simplified for purposes of this disclosure, the sclera at the posterior portion of the eye wall is designated "S" and the retina, including its uveal layer, as "R". The macula, which is centered on the optical axis, is shown as "M", and is shown in phantom lines also in Figure 9.

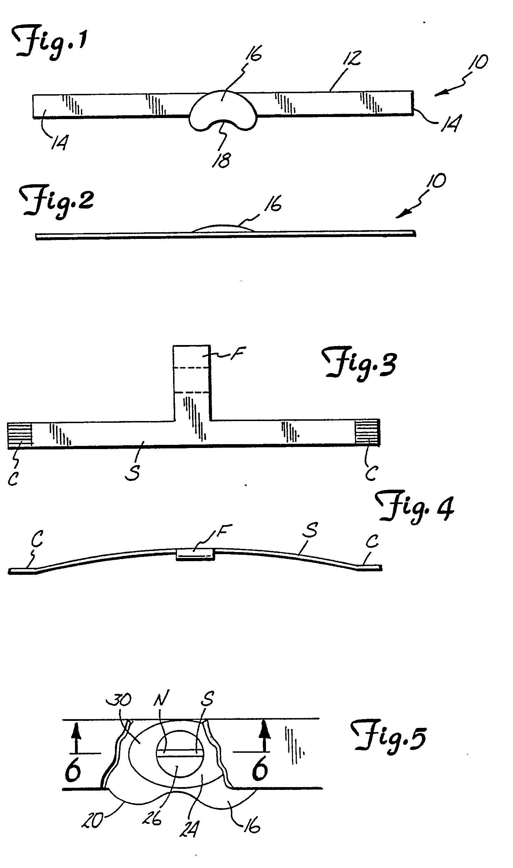

Referring now to the embodiment of Figures 1 and 2, the device of the invention is shown generally as (10) and comprises an elongated strip (12) having a length in the range of from about 50 to about 75 mm. The strip may be of any physiologically acceptable material such as donor scleral tissue or other tissue, woven polyester, silicone or siliconized fabric, and silk. The strip is flexible so as to closely conform to the outer surface of the globe of an eye. In one embodiment, the strip is inextensible; in another embodiment, the strip may be longitudinally elastically extensible. Particularly the end portions (14) of the strip are desirably suturable; that is, they are strong and tear or split-resistant so as to accept and retain sutures when the device is implanted in an eye. The strip (12) desirably is not greater than about 1.0 mm in thickness, and the longitudinal edges of the strip (12) desirably are gently rounded or are soft or non-abrasive so as to avoid unintentional irritation or damage to the eye.

A pressure pad is designated (16) and is sewn, cemented or otherwise affixed to the strip (12) intermediate the length of the strip. The pressure pad may be of any useful size and shape, edges of the pad desirably are gently rounded and soft or otherwise nonabrasive. In one embodiment, the pad (16) may be substantially incompressible in its thickness direction, although the pad may be otherwise as flexible as desired.

The term "substantially incompressible" means that the pad, when urged anteriorly against the rearward eye wall segment, will preferentially indent the eye wall rather than mainly absorb anterior pressure through compression. Some compressibility, of course, may be permitted. Since, when the device (10) is surgically positioned, the pad (16) comes into fairly close proximity with the optic nerve, the pad may be recessed along one edge as shown at (18) in Figure 1 to accommodate passage of the nerve. The pad may be so affixed to the strip as to permit adjustment of the pad along the strip to a desired position. For example, the strip may pass slidably through loops or a channel carried by the pad. Preferably, however, the pad is nonmovably attached to the strip.

As described more fully below, the surgical positioning procedure involves passing the strip (12) posteriorly about the globe of an eye and then fastening, as by sutures, the ends (14) of the strip to the sclera anteriorly of the equator of the eye and adjacent but spaced from the limbus. In this manner, the pad (16) is positioned directly behind the macula, and the forward axial pressure that is placed on the pad when the ends (14) of the strip are sutured tautly to the sclera causes the pad to push axially inwardly upon the eye wall, resulting in a slight but measurable anterior displacement, i.e., indentation, of the macula along the optical axis. The degree to which the macula may be displaced anteriorly will depend upon the tautness of the strip (12) and the thickness of the pad (16); since the tautness of the strip is limited by practical considerations involving the strength of the sutures and sutured scleral tissue, the anterior axial displacement of the macula depends largely upon the thickness of the pad (16).

In the embodiment thus described, different degrees of desired visual correction will be afforded through the use of pads having different, predetermined thicknesses and the amount of anterior pressure imparted to the pads. As shown in Figure 11, when the device of the invention is properly positioned, the pad causes axial deformation or indentation of the posterior portion of the eye wall obverse to the macula, and also may deform the strip (12) at the site of the pad (16). The correlation between pad thickness and optical correction due to anterior displacement of the macula assumes that the tension in the strip (12) and its deformation due to the presence of the pad (16) can be largely controlled. The axial displacement of the macula from the cornea may be determined with precision (within about 0.1 mm) by known ultrasound techniques at the time of surgery.

The ease of indenting the globe wall may be improved by utilizing an anterior chamber tap to release aqueous humor and thus control intraocular pressure as sutures holding the strip are drawn taut during surgery. If intraocular pressure is initially reduced, then, since the intraocular pressure will ordinarily be restored by the eye, it may be desirable to displace the macula axially forwardly a distance slightly greater than needed to correct vision since the macula will be displaced rearwardly slightly as intraocular pressure is rebuilt.

As mentioned above, the compressibility of the pad (16) in its thickness direction desirably is relatively slight and substantially less than the compressibility or deformability of the eye wall at its posterior surface obverse to the macula. That is, variations in pad thickness desirably are directly reflected in changes in the anterior displacement of the macula.

Figures 3 and 4 show the use of a strip (12) that is cut from a donor eye. Scleral tissue commonly is a somewhat fibrous tissue with the fibrous orientation running from the anterior to the posterior regions of the eye; hence, sutures that are placed in scleral tissue and that are subject to forces parallel to the fibrous orientation may cause the sclera to split or yield parallel to its fibers. Also, the strip (12) should be sufficiently long, as discussed above, as to extend from the pad (16) anteriorly about the globe of the eye for fixation to the sclera of the host eye at a point anterior to the equator of the eye and adjacent but spaced from the limbus. For this reasons, a strip of corneo-scleral material (S) may be cut from a donor eye as shown in Figure 3, the ends (C) of the strip including portions of corneal tissue. The corneal tissue is comparatively strong and tear resistant, and can be readily sutured to the sclera of the host eye. The strip of eye tissue commonly will be approximately 50—75 mm in length and desirably about 8 mm in width, and will have a thickness in the range of about 0.5—1.0 mm. A pad, either made of artificial, desirably fibrous material such as polyester or of natural donor eye tissue (as by reflecting or folding a posterior flap "F" of scleral tissue back upon itself along the dashed lines as shown in Figure 3) may be appropriately fashioned.

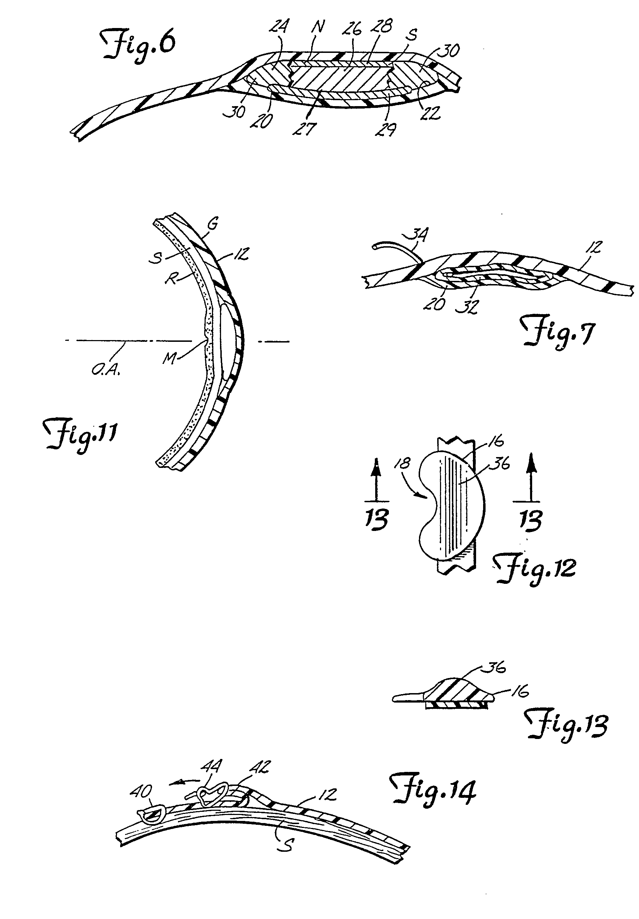

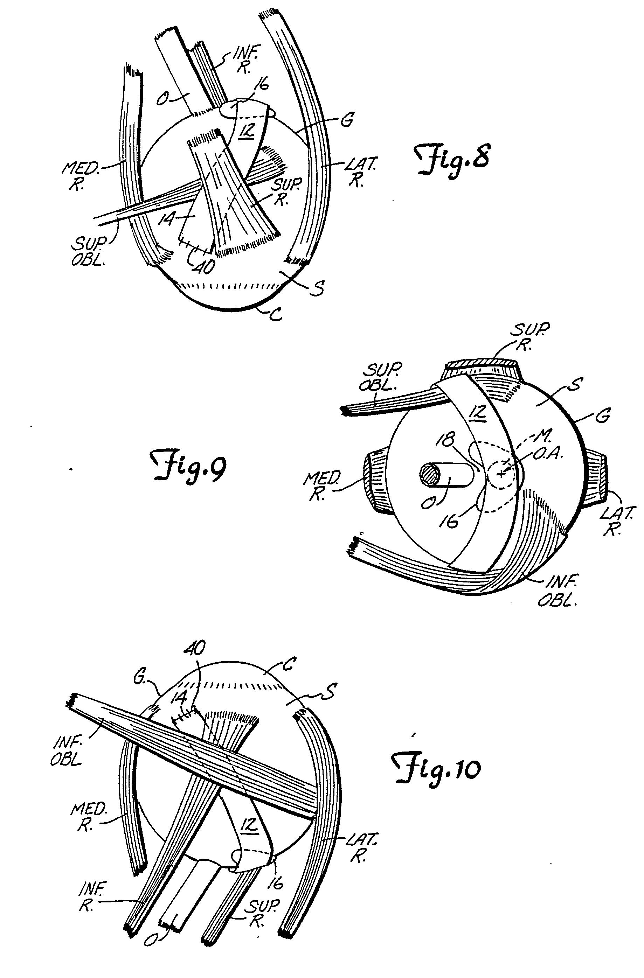

With reference to the embodiment shown in Figures 6 and 7, a web or membrane of soft, flexible material (20) such as silicone rubber may be fastened to the strip (12) to form a generally hollow cavity (22) within which may be supported an internally threaded, annular washer (24) of rigid plastic or metal or other rigid substance. A short, exteriorly threaded bolt (26) may be threadingly received in the washer, as depicted, the bolt desirably having a gently rounded surface (27) in contact with the membrane (20) or with a dished plate (29) itself in contact with the membrane (20). In this manner, as the bolt (26) is screwed inwardly or outwardly of the washer (24), the pressure of the bolt face (27) against the membrane (20) or plate (29), and hence the pressure of the membrane against the posterior portion of the eye obverse to the macula, may be adjusted. Magnetic means may be provided to permit adjustment of the position of the bolt, such means being depicted in Figures 5 and 6 as a small bar magnet (28) having north and south poles (N and S) embedded in the outer surface of the bolt (26) and having north and south poles on either side of the axis of rotation of the bolt. In this manner, once the prosthesis has been affixed to the globe, the bolt (26) may be turned through appropriate manipulation of a large magnet about the head of the recipient so that the membrane displaces the macula forwardly along the optical axis the desired distance. This feature permits the thickness of the pad to be adjusted after affixation of the prosthesis so that the most appropriate axial length of the globe is obtained. Desirably, the threads of the washer (24) or bolt (26) or both, and the other surfaces that slide past one another are appropriately lubricated to enable the bolt to be easily turned within the washer. The edges (30) of the washer may be tapered as desired to reduce sharp exterior angles.

As described more fully below with reference to Figures 12 and 13, the anterior surface of the membrane (20) or plate (29) may be contoured to provide an astigmatic correction. In the embodiment of Figures 5 and 6 in which the bolt (26) may rotate but the membrane (20) and plate (29) do not, the orientation of an astigmatic corrective contour does not change as the bolt is rotated. If desired, of course, the plate (29) may be fixed to and rotatable with the bolt so that the orientation of the astigmatic correction may be adjusted as well. It will also be understood that the bolt (26) may be affixed to the plate (29), the membrane (20) or the strip (12) to restrain it from rotation, and the threaded washer instead may be provided with magnetic poles, the axial position of the bolt (26) thus being adjusted through rotation of the washer through magnetic coupling to an external magnet.

Figure 7 shows another embodiment of the invention in which the interior of the pad is provided with an inflatable reservoir (32). Means for inflating the reservoir, typified as a small diameter tube (34), extends between the reservoir and the exterior of the device. When the prosthesis has been suitably affixed to the globe, the reservoir may be inflated with saline or other fluid through the tube (34) through the use of a hypodermic syringe or the like, the degree of inflation depending upon the desired anterior displacement of the macula. Desirably, a settable or hardenable fluid is used as the injection medium, one such fluid being a curable silicone rubber. In the latter embodiment, once the prosthesis has been affixed and the intraocular pressure has stabilized, the settable or hardenable fluid may be injected through the tube (34) into the reservoir (32) until the macula has been moved anteriorly the desired distance, following which that degree of inflation is maintained until the fluid solidifies. Exterior portions of the tube (34) can then be removed.

As mentioned above with reference to Figures 12 and 13, the surface of the pad (16) that comes into contact with the globe may be contoured to provide some astigmatic correction. As depicted in the drawing, the pad may be raised slightly in a direction normal to the optic axis to form an elongated bulge or rounded rib (36), the contour of which is transmitted to and is reflected to some extent by a similar curvature or contour of the macula. Although only a single contour change is shown in the embodiment of Figures 12 and 13, the pad may be contoured as desired.

Figures 8—10 show a preferred positioning of the prosthesis of the invention when implanted in the eye, Tenon's capsule being omitted for purposes of clarity. The device of the invention commonly may be affixed without requiring any of the eye muscles to be severed. One end of the device is passed behind the globe and over the rearward globe surface obverse to the macula and laterally of the optic nerve "O". One end of the strip (12) passes superiorly over the superior oblique muscle and under the rectus muscle and is attached at its end (14) by means of sutures (40) or the like to the sclera of the host eye. The other end (14) of the prosthesis passes inferiorly under both the inferior rectus and inferior oblique muscles, and similarly is attached to the sclera of the eye by sutures (40) or the like. To permit the tension of the strip (12) to be adjusted postoperatively (as on an out-patient basis), the strip (12) may be doubled back upon itself as shown in Figure 14 to provide an adjustable pleat, the doubled-back portion (42) being attached to the anterior portion of the strip by means of adjustable sutures (44) that can be drawn as taut as desired to provide optimum visual correction. Other tension-adjusting means, of course, may also be used.

When in place, the pad (16) is positioned directly against the eye wall obverse to the macula and is spaced laterally a short distance from the optic nerve. The recess (18) desirably formed in the pad tends to further reduce the possibility of contact between the pad and optic nerve. The strip (12) is held in place by the sutures (40) and by action of the eye muscles, particularly the superior and inferior rectus muscles and the superior and inferior oblique muscles, and the orientation of the strip against the eyeball generally follows a circumference of the eyeball passing through the point on the globe obverse to the macula. Further sutures can be added, as needed, to reduce any propensity of the strip to move once it has been correctly positioned.

Thus, the present invention provides a prosthesis which can be readily used to improve the vision of nearsighted or myopic patients by displacing the macula of the retina anteriorly along the optic axis. The degree of macula displacement, resulting from indentation of the globe obverse to the macula, may depend, in one embodiment, upon the thickness of the pressure pad that is employed or upon strip tension, and in other embodiments upon the exterior adjustment of the pressure pad thickness after the prosthesis has been implanted. The prosthesis may be used as a plumbage in aid of further surgical procedures, as in the repair of retinal holes in the posterior polar region, the latter sometimes occurring in severe diabetic and trauma cases, and may also be used to reduce posterior staphyloma (or bulging of the globe in the region of the macula) to improve the vascular supply to the posterior retinal area and to reduce mechanical damage to the retina in the macular area.

While a preferred embodiment of the present invention has been described, it should be understood that various changes, adaptations and modifications may be made therein without departing from the scope of the appended claims.

## Claims

1. A prosthesis (10) for improving the vision of myopic patients, including an elongated strip (12) of biologically acceptable material having suturable end portions (14) and which has a length ranging from about 50 to about 75 mm, and characterised by a pressure pad (16) carried by the strip (12) intermediate its length for bearing against the eye wall obverse to the macula (M) with sufficient force applied longitudinally of the optical axis (O.A.) of the eye to displace the macula longitudinally of said axis toward the cornea (C) of the eye thereby to improve the formation of an image on the macula.

2. The prosthesis of Claim 1, in which the pressure pad (16) is provided with a recess (18) along an edge thereof to accommodate passage of the optical nerve.

3. The prosthesis of Claim 1 or 2, including exteriorly operable means (24, 26, 28, 32) for varying the thickness of the pressure pad when the prosthesis has been affixed to the eye.

4. The prosthesis of Claim 3, in which said exteriorly operable means includes an internally threaded, annular washer (24) carried by the pad (16) and an exteriorly threaded bolt (26) threadingly received in the washer for movement, upon rotation thereof, in a direction normal to the length of the strip (12), and magnetic means (28) carried by the bolt enabling the latter to be rotated through manipulation of a magnetic field exterior of the eye of a patient.

5. The prosthesis of Claim 4, wherein said pressure pad includes a nonrotatable, anterior facing membrane (20) to receive axial pressure from said bolt (26) and transmit such pressure to the eye wall.

6. The prosthesis of Claim 3, wherein said exteriorly operable means comprises an inflatable reservoir (32) carried by the pad, and means (34) enabling inflation of the reservoir externally of the eye.

7. The prosthesis of any of the preceding claims, wherein said pad has a surface contactable with the globe of the eye obverse to the macula, said surface having a predetermined asymmetric contour (36) to provide astigmatic correction.

8. The prosthesis of any of the preceding claims, wherein the strip is longitudinally substantially inelastic.

9. The prosthesis of any of Claims 1 to 7 wherein the strip is longitudinally elastic.

10. The prosthesis of any of the preceding claims, wherein the pad is substantially incompressible in its thickness direction.

11. The prosthesis of any of the preceding claims, in which the intermediate portion of the strip and the pad are resiliently curved so as to fit snugly against the posterior wall of the globe of the eye obverse to the macula.

## Patentansprüche

1. Prothese (10) zur Verbesserung der Sehkraft von kurzsichtigen Patienten, welche einen langgestreckten Streifen (12) aus biologisch verträglichem Material aufweist mit vernähbaren Endabschnitten (14) und einer Länge zwischen etwa 50 und 75 mm, gekennzeichnet durch ein Druckpolster (16), welches von dem Streifen (12) intermediär seiner Länge zum Tragen gegen die Augenwand gegenüberliegend der Makula (M) gehalten ist und zwar mit genügender Kraft in Längsrichtung der optischen Achse (O.A.) des Auges, um die Makula in Längsrichtung dieser Achse zur Cornea (C) des Auges hin zu verschieben und damit die Erzeugung einer Abbildung auf der Makula zu verbessern.

2. Prothese nach Anspruch 1, dadurch gekennzeichnet, daß das Druckpolster (16) längs seines einen Randes mit einer Vertiefung (18) zur Anpassung an den Durchgang des Sehnervs versehen ist.

3. Prothese nach Anspruch 1 oder 2, gekennzeichnet durch äußere betätigbare Mittel (24, 26, 28, 32) zum Verändern der Dicke des Druckpolsters, nachdem die Prothese am Auge befestigt worden ist.

4. Prothese nach Anspruch 3, dadurch gekennzeichnet, daß die äußeren betätigbaren Mittel eine im Innern mit einem Gewinde versehene, ringförmige Scheibe (24) aufweisen, welche von dem Polster (16) getragen ist, eine außen mit einem Gewinde versehenen Bolzen (26), welche einschraubbar in der Scheibe aufgenommen ist für eine Bewegung in eine Richtung senkrecht zu der Länge des Streifens (12) bei Verdrehen, und magnetische Mittel (28), welche durch den Bolzen getragen sind und letzteren zu einer Rotation durch Beeinflussung eines magnetischen Feldes außerhalb des Auges des Patienten veranlassen.

5. Prothese nach Anspruch 4, dadurch gekennzeichnet, daß das Druckpolster eine nichtverdrehbare, nach vorne gerichtete Membran (20) aufweist, um axialen Druck von dem Bolzen (26) zu erhalten und diesen Druck an die Augenwand weiterzuleiten.

6. Prothese nach Anspruch 3, dadurch gekennzeichnet, daß die äußeren betätigbaren Mittel ein aufblähbares Reservoir (32) aufweisen, welches von dem Polster getragen ist, und Mittel (34), welche ein Aufblähen des Reservoirs von außerhalb des Auges ermöglichen.

7. Prothese nach einem der vorhergehenden Ansprüche, dadurch gekennzeichnet, daß das Polster eine zur Berührung mit dem Augapfel gegenüberliegend der Makula ausgebildete Oberfläche aufweist, daß die Oberfläche eine vorbestimmte asymmetrische Kontur (36) aufweist, um eine astigmatische Korrektur zu ermöglichen.

8. Prothese nach einem der vorhergehenden Ansprüche, dadurch gekennzeichnet, daß der Streifen in Längsrichtung im wesentlichen unelastisch ist.

9. Prothese nach einem der Ansprüche 1 bis 7, dadurch gekennzeichnet, daß der Streifen in Längsrichtung elastisch ist.

10. Prothese nach einem der vorhergehenden Ansprüche, dadurch gekennzeichnet, daß das Druckpolster in Richtung seiner Dicke im wesentlichen inkompressibel ist.

11. Prothese nach einem der vorhergehenden Ansprüche, dadurch gekennzeichnet, daß der intermediäre Abschnitt des Streifens und das Polster elastisch gekrümmt sind, so daß sie fest gegen die hintere Wand des Augapfels gegenüberliegend der Makula anliegen.

**Revendications**

1. Prothèse (10) pour améliorer la vue de patients myopes, comportant une bande allongée (12) faite en un matériau biologiquement acceptable, qui possède des parties d'extrémité (14) aptes à être suturées et une longueur comprise dans une plage allant d'environ 50 à environ 75 mm, et caractérisée par un coussinet de pression (16) porté par la bande (12) en un point intermédiaire de sa longueur en vue d'appuyer contre la paroi oculaire, à l'opposé de la macula (M), une force suffisante étant appliquée longitudinalement à l'axe optique (O.A.) de l'oeil pour déplacer la macula longitudinalement audit axe en direction de la cornée (C) de l'oeil, pour ainsi améliorer la formation d'une image sur la macula.

2. Prothèse de la revendication 1, dans laquelle le coussinet de pression (16) présente un creux (18) disposé le long d'un bord de celui-ci pour permettre le passage du nerf optique.

3. Prothèse de la revendication 1 ou 2, comportant des moyens (24, 26, 28, 32) pouvant être actionnés de l'extérieur pour faire varier l'épaisseur du coussinet de pression, une fois que la prothèse a été apposée sur l'oeil.

4. Prothèse de la revendication 3, dans laquelle lesdits moyens pouvant être actionnés de l'extérieur comportent une rondelle annulaire (24) filetée intérieurement et portée par le coussinet (16), et un boulon (26) fileté extérieurement et logé par vissage dans la rondelle en vue d'être déplacé, lors d'une rotation de celle-ci, dans une direction perpendiculaire à la longueur de la bande (12), ainsi qu'un moyen magnétique (28) porté par le boulon et permettant une rotation de ce dernier grâce à une manipulation d'un champ magnétique extérieur à l'oeil d'un patient.

5. Prothèse de la revendication 4, dans laquelle ledit coussinet de pression comporte une membrane (20) tournée vers l'avant, immobilisée en rotation et destinée à recevoir une pression axiale exercée par ledit boulon (26) et à transmettre cette pression à la paroi oculaire.

6. Prothèse de la revendication 3, dans laquelle lesdits moyens pouvant être actionnés de l'extérieur comportent un réservoir gonflable (32) porté par le coussinet, et un moyen (34) permettant de gonfler le réservoir extérieurement à l'oeil.

7. Prothèse de l'une quelconque des revendications précédentes, dans laquelle ledit coussinet possède une surface apte à venir en contact avec le globe de l'oeil, à l'opposé de la macule, ladite surface ayant un contour asymétrique prédéterminé (36) afin d'assurer une correction astigmatique.

8. Prothèse de l'une quelconque des revendications précédentes, dans laquelle la bande est sensiblement non-élastique dans le sens de la longueur.

9. Prothèse de l'une quelconque des revendications 1 à 7, dans laquelle la bande est élastique dans le sens de la longueur.

10. Prothèse de l'une quelconque des revendications précédentes, dans laquelle le coussinet est sensiblement incompressible dans le sens de son épaisseur.

11. Prothèse de l'une quelconque des revendications précédentes, dans laquelle la partie intermédiaire de la bande et le coussinet sont courbés élastiquement de façon à s'ajuster étroitement contre la paroi postérieure du globe de l'oeil, à l'opposé de la macula.

0 138 564

Fig.1

16    12    10

14    18    14

Fig.2    16    10

Fig.3

F

C    S    C

Fig. 4

C    F    S    C

Fig.5

30    N    S

6    6

20    26    24    16

1

*Fig.6*

24 N 26 28 S 30

30 20 27 29 22

*Fig.11*

G

S

12

R

O.A. M

*Fig.7*

34 12

20 32

*Fig.12*

16

18 36

13 13

*Fig.13*

36

16

*Fig.14*

44 42

40 12

S

Fig.8

Fig.9

Fig.10